(19) Europäisches Patentamt
European Patent Office
Office européen des brevets

(11) Publication number: **0 135 297**
**A2**

# (12) EUROPEAN PATENT APPLICATION

(21) Application number: 84304915.6

(22) Date of filing: 18.07.84

(51) Int. Cl.[4]: C 12 P 7/40
C 07 J 1/00, C 07 J 9/00
C 12 N 1/20
//(C12N1/20, C12R1:06)

(30) Priority: 19.07.83 JP 132092/83

(43) Date of publication of application:
27.03.85 Bulletin 85/13

(84) Designated Contracting States:
CH DE FR GB LI

(71) Applicant: SHIONOGI & CO., LTD.
12, Dosho-machi 3-chome Higashi-ku
Osaka 541(JP)

(72) Inventor: Kondo, Eiji
4-22-18, Ishibashi
Ikeda-shi Osaka(JP)

(72) Inventor: Mitsugi, Takashi
2-15-27, Kitatoyonaka-cho
Izumiotsu-shi Osaka(JP)

(74) Representative: Bizley, Richard Edward et al,
BOULT, WADE & TENNANT 27 Furnival Street
London EC4A 1PQ(GB)

## (54) Synthesis of indanedionepropionic acids and products therefrom, microorganisms for use therein and their production.

(57) A useful intermediate, 7aβ-methyl-1,5-dioxo-3aα-hexahydroindane-4α-propionic acid, may be prepared from 3-(hydroxy or oxo)steroids by the action of mutant strains of the genus *Arthobacter simplex* which lack the metabolic system for assimilating the objective acid. The invention includes such mutant strains and their preparation.

EP 0 135 297 A2

0135297

# SYNTHESIS OF INDANEDIONEPROPIONIC ACIDS AND PRODUCTS THEREFROM, MICROORGANISMS FOR USE THEREIN AND THEIR PRODUCTION

This invention relates to the microbial production of 7aβ-methyl-1,5-dioxo-3aα-hexahydroindane-4α-propionic acid (hereinafter referred to as "indanedionepropionic acid" for simplicity) represented by Formula (I) below and starting from a 3-(hydroxy or oxo) steroid.

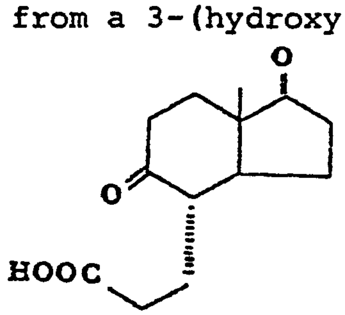

(I)

Indanedionepropionic acid (I) is a known intermediate for preparing 19-norteroids, antiandrogens, and the like. Microbial production of this compound is also known from e.g., Japanese patent application Kokai 53-69885 and Biochimica et Biophysica Acta, Vol. 531, page 308 (1978), using Mycobacterium fortuitum NRRL B-8129 for converting a sterol to a Compound I. However, this prior art method is not practical as its yield is too low.

The present invention is based upon the discovery that by treating Arthrobacter simplex IFO 3530 with N-methyl-N'-nitro-N-nitrosoguanidine a mutant is obtained in which the metabolic system for assimilating indanedionepropionic acid is absent. This may be applied to treat a 3-(hydroxy or oxo)steroid to obtain a compound (I) in high yield.

Thus, this invention provides, in one aspect, a process for the production of indanedionepropionic acid (I), which process comprises permitting a strain of Arthrobacter simplex lacking the metabolic system for assimilating indanedionepropionic acid (I) to act upon a 3-(hydroxy or oxo)steroid.

The starting 3-(hydroxy or oxo)steroid used in this invention may be, e.g., cholesterol, sitosterol, campesterol, 1,4-androstadien-3,17-dione, 4-androsten-3,17-dione, testosterone, progesterone, or a cholic acid.

The microorganism used in this invention is a mutant of Arthrobacter simplex lacking the metabolic system for assimilating indanedionepropionic acid (I). The invention includes such mutants. Representative of such mutant strains is the strain which may be derived from Arthrobacter simplex IFO 3530 by the use of N-methyl-N'-nitro-N-nitrosoguanidine. This strain is named Arthrobacter simplex ID-38-6 and is on deposit at the

Institute of Fermentation Technology, Japan, with Deposit No. BP-260. The deposit is in accordance with the Budapest Treaty and was made on 2nd March 1983.

The above new mutant strain is a chemical variant of Arthrobacter simplex IFO 3530, but its morphological and nutritional characteristics are believed to be the same as those of the parent strain. The main taxonomic characteristics of Arthrobacter simplex IFO 3530 (and ID-38-6) are as follows:

[A] Morphology and stainability -

1. Vegetative cells (Gly-IM agar and nutrient agar, 28°C, 1-8 days): Short rods to coccoidal cells, 0.4-0.6 x 0.8-3.0μ, are observed at 1 day stage. All cells become coccoid, 0.5-0.6 x 0.6-0.8μ, at 2 days or a later stage. Beaded arrangement is observed at coccoid stage.

2. Gram staining (Nutrient agar, 28°C, 1 to 8 days): Positive.

3. Spore: None.

[B] Growth Characteristics -

1. Colony on agar (Nutrient agar, 28°C, 4 days): Circular 0,3 to 1.5 mm, convex, entire colonies with shining smooth surface. Semitransparent density, soft to butyrous structure and grayish white color.

2. Agar stroke (Nutrient agar, 1-2 days): Moderate filiform growth with shining surface. Grayish white

color, semitransparent density and soft to butyrous structure.

3. Broth (Gly-1M medium, 28°C, 1-10 days): Moderate, uniform growth is seen in the early stage. Some precipitation occurs at a later stage.

[C] Conditions for Growth -

1. Oxygen requirement (Gas Pak System): Aerobic.

2. Temperature for growth (Multi-temperature incubator, Toyo scientific industries Co., Ltd. Tokyo): Good growth is seen at 18.5°C to 37°C. The most rapid growth is observed at 27°C to 35.5°C. On agar slant, visible growth is seen at 37°C.

3. pH for growth (Gly-IM medium, 28°C, 1 day): Good growth is seen at pH 6.28 to 10.1. The best growth is observed at pH 7.42 to 8.10.

[D] Physiological characteristics -

1. Catalase: Positive.

2. Oxidase (Kovac's): Positive.

3. Nitrite formation from nitrate: Positive.

4. Gelatin liquefaction: Positive.

5. Starch hydroylis: Negative.

6. Nutritional requirements: No requirement.

[E] Chemical Composition of Cells -

1. Amino acid composition in cell wall: DAP, Glu and Ala are detected as major amino acids of cell wall peptido-glycan.

2. Sugar composition of cell wall: Galactose, Rhamnose and Glucosamine are detected as major carbohydrates of cell wall peptidoglycan.

3. Hydroxy fatty acids (Mycolic acids) in vegetative cells: Mycolic acids are detected in the cells.

The invention includes not merely Arthrobacter simplex ID-38-6 but also mutants or variants thereof possessing the ability to produce indanedionepropionic acid (I) as above and lacking the capability of assimilating the aforesaid acid.

Of course, such mutants of Arthrobacter simplex ID-38-6 may be deliberately produced, if desired, by standard mutagenic and isolation techniques and need not be naturally occurring. Although not limited thereto, such mutants may have the characteristics of Arthrobacter simplex.

In performing the process of the invention, a seed culture may be produced by inoculating such a mutant of Arthrobacter simplex into a nutrient medium. This may then be inoculated into a medium containing the starting steroid, the substrate, and propagated in a conventional manner. The thus-cultured broth may be freed from the starting material by, e.g., solvent extraction, acidification of the water layer, extraction in a conventional manner, concentration, and purification in a normal manner

to obtain the objective indanedionepropionic acid (I).

In such production, the steroid concentration is preferably from 0.1 to 0.2 w/v %. In this concentration region, the yield of indanedionepropionic acid (I) is usually about 85 to 97%.

Naturally, the invention also includes a process for producing a mutant of the invention which comprises treating Arthrobacter simplex IFO 3530 with N-methyl-N'-nitro-N-nitrosoguanidine as a mutagenic agent.

The following Examples further illustrate this invention.

Example 1

A nutrient aqueous solution (10 ml) containing Bacto Nutrient Broth (a Trade name of Difco Corp.) (0.08 g) and Bacto Yeast Extract (a Trade name of Difco Corp.) (0.01 g) in deionized water is adjusted to pH 7.0 with 1N-sodium hydroxide, placed in a test tube, and sterilized with steam at 120°C for 15 minutes. Into this solution is inoculated a loopful of culture of Arthrobacter simplex IFO 3530 cells on 20 % potato agar slant medium prepared by propagating at pH 7.0 at 28°C for 3 days. After shaking at 28°C for 20 hours, the thus-obtained seed culture (1 ml) is inoculated into a liquid medium (10 ml) of the same composition, and cultivated for 4 hours at 28°C. Then, the cells are collected by centrifugation at 1200G for 10 minutes and washed with a M/20 disodium hydrogen phosphate - potassium dihydrogen phosphate buffer (pH 7.0).

The cells are suspended in a buffer of the same composition to make about 4.4 x $10^9$ cells per milliliter, mixed with N-methyl-N'-nitroso-N-nitroguanidine in an amount corresponding to 1000 μg of the substance per milliliter of the buffer, and shaken at 28°C for 40 minutes. The cells in the suspension are repeatedly washed with physiological saline water by centrifugation, suspended in the same solution, and then distributed on an agar nutrient medium (Nissui Seiyaku) in petri dishes (9 cm in diameter). These are cultured at 28°C for 3 days, and the colonies which appear are replicated in two kinds of agar plates made of minimum nutrient medium. The two kinds of plates have the following constituents:

Minimum Agar Medium (pH 7.0)

| Component | Weight (g/l) |
|---|---|
| Carbon source | 1.00 |
| Ammonium sulfate | 2.00 |
| Potassium dihydrogen phosphate | 1.00 |
| Potassium chloride | 0.50 |
| Magnesium sulfate heptahydrate | 0.50 |
| Ferrous sulfate heptahydrate | 0.10 |
| Agar Purified (Difco Corp) | 17.00 |
| Water to make | 1 liter |

One has sodium acetate and the other has 7aβ-methyl-1,5-dioxo-3aα-hexahydroindane-4α-propionic acid sodium salt as a

sole carbon source. Judging from the colony growth of the replicas, a mutant strain lacking the ability of assimilating indanedionepropionic acid is isolated and designated as Arthrobacter simplex ID-38-6.

Example 2

An aqueous solution (100 ml) as a seed culture medium containing polypeptone (0.5 g), corn steep liquor (0.5 g), and sodium acetate (0.1 g) in deionized water is adjusted to pH 7.0 with aqueous 1N-sodium hydroxide, placed in a 500 ml flask, and sterilized with steam at 120°C for 15 minutes. Then one loopful of Arthrobacter simplex ID-38-6 obtained by the method of Example 1 is inoculated into the solution and shaken for 41 hours at 28°C to give a seed culture. An aqueous solution (200 ml) as a liquid culture medium containing ammonium sulfate (0.4 g), potassium dihydrogen phosphate (0.2 g), potassium chloride (0.1 g), magnesium sulfate heptahydrate 0.1 g ferrous sulfate heptahydrate (0.002 g), and sodium acetate (1 g) in deionized water is adjusted to pH 7.0 with aqueous 1N-sodium hydroxide, placed in two 500 ml flasks, sterilized with steam at 120°C for 15 minutes, and mixed with cholesterol powder (0.15 g each flask) pulverised at 120 mesh or more. This medium is inoculated with the above seed culture (10 ml each flask) and shaken at 28°C for 120 hours. Then whole culture broth (pH 8.6) is washed with ethyl acetate (200 ml), acidified with 2N-hydrochloric acid to pH 1.8, and extracted

with ethyl acetate (3 x 200 ml). The extract is concentrated in vacuum to obtain 200 mg of residue. This is dissolved in dichloromethane, filtered to remove solid, and concentrated. The residue is crystallized from ethyl ether to give indanedionepropionic acid (I) (161.0 mg; Yield: 87.1 %). A sample recrystallized from ethyl ether gives the following physicochemical characteristics and is identified with an authentic sample.

m.p. 108 - 108.5°C.

IR $\nu(CHCl_3)$: 3600-2400, 1745, 1711 $cm^{-1}$.

$[\alpha]_D^{24}$ 97.3° ± 1.4° (c = 1.009, $CHCl_3$).

NMR $\delta(CDCl_3)$: 1.17 (3H, s) ppm.

Elemental Analysis:

Calcd. for $C_{13}H_{18}O_4$ : C, 65.53; H, 7.61

Found : C, 65.25; H, 7.64

Example 3

An aqueous solution (400 ml) as a liquid culture medium containing ammonium sulfate (10 g), potassium dihydrogen phosphate (0.5 g), potassium chloride (0.25 g), magnesium sulfate heptahydrate (0.25 g), ferrous sulfate heptahydrate (0.005 g), sodium acetate (2.5 g), and Bacto-Soytone (Trade name of Difco Corp) (2.5 g) in deionized water is adjusted to pH 7.0 with aqueous sodium hydroxide, poured into five 500 ml flasks (80 ml each) and sterilized with steam at 120°C for 15 minutes. A suspension of cholesterol (6 g), Tween 20

(polyethylenesorbitan monolaurate) (1 g), and Span 80 (sorbitan monooleate) (1 g) in sterile distilled water is diluted with water (100 ml) and homogenized for 5 minutes with 150 W Hypersonic homogenizer (20 kHz). Twenty milliliter of the suspension is added to the liquid medium in each flask. Then 10 ml of the seed culture medium of Example 2 is added to each flask and the mixture is shaken at 28°C for 96 hours. The combined culture broth (pH 6.9) is brought to pH 9.0 with 1N-sodium hydroxide and washed with ethyl acetate (500 ml). The aqueous layer is acidified with 2N-hydrochloric acid to pH 2 and extracted with ethyl acetate (3 x 500 ml). The combined extract is concentrated in vacuum to give a residue (3.7 g). This is dissolved in dichloromethane, filtered to remove solid, and concentrated. The residue is crystallized from ethyl ether to give indanedionepropionic acid (I) (2.863 g). Yield: 77.4 %.

Example 4

An aqueous solution (200 ml) as a liquid culture medium containing ammonium sulfate (0.4 g), potassium dihydrogen phosphate (0.2 g), potassium chloride (0.1 g), magnesium sulfate heptahydrate (0.1 g), ferrous sulfate heptahydrate (0.002 g), and sodium acetate (0.2 g) in deionized water is adjusted to pH 7.0 with aqueous 1N-sodium hydroxide, poured into two 500 ml flasks (100 ml each) and sterilized with steam at 120°C for 15 minutes. Androst-4-en-3,17-dione powder (0.2 g each) is added to each flask. After the

seed culture of Example 2 (5 ml each) is added to the medium, the mixture is shaken at 28°C for 23 hours. The culture broth of the flasks (200 ml) is then combined, acidified with 2N-hydrochloric acid to pH 1.8, and extracted with ethyl acetate (3 x 200 ml). The extract is treated in a manner similar to that of Example 3 to give crystalline indanedionepropionic acid (I) (320 mg; Yield: 96.2 %). This is treated with active charcoal in dichloromethane and recrystallized from ethyl ether to give a pure product (269.0 mg).

m.p. 109°C.

IR ν($CHCl_3$): 3600-2400, 1745, 1711 $cm^{-1}$.

$[\alpha]_D^{24}$ 99.7° ± 1.4° (c = 1.015, $CHCl_3$).

NMR δ($CDCl_3$): 1.17 (3, 3H)ppm.

Mass (m/e): 238 ($M^+$)

Elemental Analysis:

Calcd for $C_{13}H_{18}O_4$ : C, 65.53; H, 7.61

Found : C, 65.59; H, 7.66

Example 5

The method of Example 2 is repeated substituting cholesterol by powdered progesterone (0.4 g), the amount of liquid culture medium by 100 ml, the medium by one containing Tween 20 (0.5 g), and the shaking time by 96 hours. The broth is treated in a manner similar to that of Example 2 substituting ethyl acetate extraction with that using thrice 100 ml, to give indanedionepropionic acid (I) (137.0 mg; Yield 45.2 %).

Example 6

The method of Example 5 is repeated substituting for the substrate progesterone beta-sitosterol powder of higher than 100 mesh (0.4 g) and the cultivation time by 168 hours. The ethyl acetate extract is chromatographed over a thin layer of silica gel developing with a mixture of cyclohexane : ethyl acetate : acetic acid (10 : 10 : 2 volume ratio). Coloring by spraying concentrated sulfuric acid and heating gives a spot of indanedionepropionic acid (I) at Rf 0.37.

Example 7

An aqueous solution (100 ml) containing polypeptone (0.05 g), corn steep liquor (0.05 g), sodium acetate (0.01 g), and and sodium cholate (0.01 g) in deionized water is adjusted to pH 7.0 with aqueous 1N-sodium hydroxide, poured into a test tube, sterilized with steam at 120°C for 15 minutes, inoculated with a seed culture of Example 2 (0.5 ml), and shaken at 28°C for 2 days. The thus-obtained culture broth is acidified to pH 1.8 with 2N-hydrochloric acid and extracted with ethyl acetate. The extract is subjected to thin-layer chromatography to give spots of indanedionepropionic acid at Rf 0.37 and (4R)-4-[4α-(2-carboxyethyl)-7aβ-methyl-5-oxo-3aα-hexahydroindan-1β-yl]valeric acid of the following formula (II) at Rf 0.52.

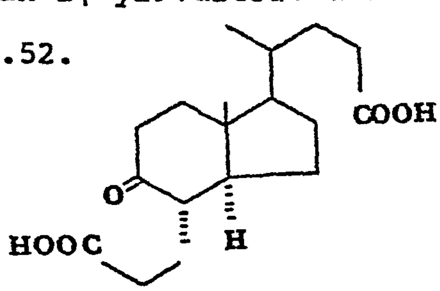

(II)

CLAIMS:

1. A process for preparing 7aβ-methyl-1,5-dioxo-3aα-hexahydroindane-4α-propionic acid, which process comprises treating a 3-(hydroxy or oxo)steroid with a strain of the genus Arthrobacter simplex lacking the metabolic system for assimilating the objective compound.

2. A process as claimed in claim 1, wherein the starting 3-(hydroxy or oxo)steroid is cholesterol, sitosterol, campesterol, 1,4-androstadien-3,17-dione, 4-androsten-3,17-dione, testosterone, progesterone, or a cholic acid.

3. A process as claimed in claim 1 or claim 2, wherein the steroid concentration is from 0.1 to 0.2 w/v %.

4. A process as claimed in any one of claims 1 to 3, wherein the strain used is Arthrobacter simplex ID-38-6.

5. A mutant of Arthrobacter simplex lacking the metabolic system for assimilating 7aβ-methyl-1,5-dioxo-3aα-hexahydroindane-4α-propionic acid.

6. Arthrobacter simplex ID-38-6 or a mutant or variant thereof possessing the ability to produce 7aβ-methyl-1,5-dioxo-3aα-hexahydroindane-4α-propionic acid from a 3-(hydroxy or oxo)steroid but lacking the capability of assimilating the same acid.

7. A process for producing a mutant as claimed in

claim 5 which comprises treating <u>Arthrobacter simplex</u> <u>IFO 3530</u> with N-methyl-N'-nitro-N-nitrosoguanidine as a mutagentic agent.

8. A 19-norteroid or antiandrogen which has been produced from 7aβ-methyl-1,5-dioxo-3aα-hexahydroindane-4α-propionic acid made by a process as claimed in any one of claims 1 to 4.